# NEUE EUROPÄISCHE PATENTSCHRIFT

(11) **EP 2 388 311 B2**
(45) Veröffentlichungstag und Bekanntmachung des Hinweises auf die Entscheidung über den Einspruch: **18.12.2019**
(45) Hinweis auf die Patenterteilung: 30.03.2016
(21) Anmeldenummer: 10450088.9
(22) Anmeldetag: 19.05.2010
(51) Int. Cl.: C12M 1/107, F17B 1/26

(54) **Gasspeicher**
Gas storage device
Réservoir de gaz

(43) Veröffentlichungstag der Anmeldung: 23.11.2011
(73) Patentinhaber: Sattler AG, 8041 Graz (AT)
(72) Erfinder: Wiedau, Helmut, 48161 Münster (DE)
(74) Vertreter: Ellmeyer, Wolfgang

(56) Entgegenhaltungen:
- EP-A1- 0 045 114
- EP-A1- 0 045 114
- EP-A1- 1 801 037
- EP-A2- 1 338 843
- EP-A2- 1 647 760
- EP-A2- 1 647 760
- WO-A1-83/03884
- WO-A1-2009/155629
- DE-A1- 10 115 623
- DE-A1- 10 115 623
- DE-A1- 10 302 658
- DE-A1- 19 650 055
- DE-A1-102007 005 069
- DE-A1-102007 005 069
- US-A- 4 902 304
- US-A- 4 902 304
- Barbara Eder; Heinz Schulz: "Biogas-Praxis, 3. vollständig überarbeitete und erweiterte Auflage 2006", 2006 ISBN: 3-936896-13-5
- Schriftliche Erklärung unter Eid von Josef Baur zur Vorbenutzung "Krattenweiler"
- Schriftliche Erklärung unter Eid von Josef Baur zur Vorbenutzung "Bahra"
- Interner Fertigungsauftrag für das Gasspeicherkissen "Krattenweiler" vom 24. Juni 2006
- Rechnung über das Gasspeicherkissen "Krattenweiler" an die Bioenergie Sauter GbR vom 25. Juli 2006
- Zeichnungssatz Genehmigungsplanung "Bahra" vom Februar 2007, 3 Blatt
- Lieferschein Folien "Bahra" vom 30. August 2007
- Rechnung Folien "Bahra" vom 8.Juli 2008
- Modellkizze "Bahra"

## Beschreibung

Die Erfindung betrifft einen Gasspeicher mit einem Speicherboden und einer Begrenzungswand zur Aufnahme von Substrat, aus dem durch Fermentation Biogas erzeugbar ist, sowie einer gasdichten Abdeckung mit zumindest zwei, an ihrem Rand fixierten, gasdichten Membranen, die eine Innen- und eine Außenmembran umfassen, wobei die zumindest zwei gasdichten Membranen so konfektioniert sind, dass sie im entleerten Zustand des Gasspeichers zumindest teilweise auf dem Speicherboden und der Begrenzungswand flächig aufliegen.

Als Gasspeicher werden im Rahmen der Erfindung alle Behälter bezeichnet, die in der Lage sind, Gas zu speichern, unabhängig davon ob das zu speichernde Gas innerhalb dieses Behälters erzeugt oder von außen zugeleitet wird.

Bei der Erzeugung von Biogas, insbesondere bei der Fermentation, der Nachgärung und der Endlagerung spielt die Speicherung des Biogases eine bedeutende Rolle.

Besonders die Veränderbarkeit des Gasspeichervolumens zur Anpassung an die Schwankungen bei der Gaserzeugung stellt eine wichtige Anforderung dar, die durch Membran-Gasspeicher erfüllt wird. Eine Gasspeicherfolie bzw. -membran, sie kann aus verschiedenen Materialien bestehen, z.B. aus LDPE, PP, PP/PVC oder allgemein einem beschichteten Gewebe, wird durch Klemmung an einer Behälterwand eingespannt, welche den Innenraum umgibt, in dem die Fermentation z.B. von bei der Nutztierhaltung anfallenden Ausscheidungen durchgeführt wird. Das entstehende oder eingespeiste Biogas füllt den gasdichten Innenraum, sodass sich die Speichermembran entsprechend ausweitet.

Es kann auch eine zweischalige gasdichte Abdeckung, wie z.B. bei einem Doppelmembran-Biogasspeicher vorgesehen sein, bei der sich die Innenmembran je nach Gasvolumen hebt und senkt und von der sie umgebenden Außenmembran geschützt wird. Durch Veränderung des zwischen der Innen- und Außenmembran wirkenden Stützdruckes wird die Form der Außenmembran weitgehend konstant gehalten, während die Innenmembran entsprechend ihrer Befüllung sich ausdehnen oder zusammenziehen kann. Die Außenmembran nimmt sämtliche Belastungen durch Schnee, Wind und andere Witterungseinflüsse auf.

Die Innenmembran bildet somit die gasdichte Abdeckung des Gasspeichers. Der Druck im Stützluftraum zwischen der Innenmembran und der Außenmembran überträgt sich über die Innenmembran auf das gespeicherte Biogas. Je nach Füllstand schwebt daher die Innenmembran auf unterschiedlicher Höhe zwischen einem im Inneren des Gasspeichers angebrachten Gurtsystem und der Außenmembran.

Das Gurtsystem verhindert durch mehrere im Wesentlichen horizontal gespannte Gurten, dass sich die Innenmembran bei übermäßiger Entleerung des Gasspeichers bis zum Substrat absenkt und durch Rührwerke beschädigt oder durch Substratreste verschmutzt wird.

Allerdings ist die Errichtung eines entsprechenden Gurtsystems mit einem hohen Aufwand verbunden, da das Gurtsystem die gesamte statische Last des Membrangewichts und in Abhängigkeit von der Witterung auch beträchtliche Schnee- und/oder Wasserlasten abzutragen hat.

Zudem kann es bei einem Gasspeicher mit Gurtsystem bei einer vollständigen Entleerung des Gasspeichers zur Entstehung eines Unterdrucks kommen, weil die Speichermembran nicht vollständig zu Boden sinken kann. Dies führt dann zur Auslösung der Unterdrucksicherung, was an sich nicht erwünscht ist, weil dann Luft bzw. Sauerstoff in den Gasspeicher gelangen kann.

Aus der DE 103 02 658 A1 geht eine Trockenfermentationsanlage hervor, bei der der Fermentationsraum mit einem beheizten Gasmantel umgeben ist, der zum Fermentationsraum eine gasdichte wäremübertragende Wandung aufweist. Es sind dabei eine Außenmembran und eine Innenmembran vorgesehen, die eine trockene Materialschüttung abdecken, wobei die Innenmembran am höchsten Punkt der trockenen Materialschüttung aufliegt. Beim Entleeren oder Befüllen der Trockenfermentationsanlage werden, sofern keine Stützkonstruktion vorgesehen ist, Außen- und Innenmembran abgenommen, um die Miete zu entnehmen oder diese einzubringen. Ein Betriebszustand mit abgesenkter Innen- und Außenmembran existiert dabei nicht.

Aufgabe der Erfindung ist es daher, einen Gasspeicher der eingangs genannten Art anzugeben, der ohne Gurtsystem auskommt und dennoch ein sicheres und geschütztes Ablegen der Membran ermöglicht, ohne dass äußere Einflüsse oder scharfe Kanten oder Maschinenteile eine Beschädigung der Membran hervorrufen.

Erfindungsgemäß wird dies dadurch erreicht, dass der Gasspeicher eine Substratleitung umfasst, über die im Betrieb Substrat in den Gasspeicher geleitet werden kann.

Die zumindest eine gasdichte Membran kann somit während des Entleervorganges vollständig auf die Boden- oder Wandfläche des erfindungsgemäßen Gasspeichers absinken und wird von dieser getragen. Alle witterungsbedingten oder sonstige Lasten werden ebenfalls auf diese Weise aufgenommen. Auf das sonst übliche Gurtsystem kann verzichtet werden, wodurch es beim Entleeren des erfindungsgemäßen Gasspeichers nicht zum Ansprechen der Unterdrucksicherung kommen kann.

Die Membran wird gemäß einer weiteren Ausführungsform der Erfindung so dimensioniert, dass zumindest entlang einem Querschnitt durch den Gasspeicher die Bogenlänge b der zumindest einen Membran im aufgespannten Zustand größer oder gleich der zwischen gegenüberliegenden Einspannpunkten der zumindest einen gasdichten Membran gemessenen Länge der Durchlauflinie d entlang dem Speicherboden und gegebenenfalls der Begrenzungswand ist.

Das auf diese Weise definierte Maß kann z.B. bei kreisförmigem Grundriss des erfindungsgemäßen Gasspeichers anhand eines Querschnittverlaufes des Gasspeichers ermittelt werden.

Es können aber Behälter jeglicher Art in erfindungsgemäßer Weise ohne Gurtsystem abgedeckt werden, sowohl runde zylindrische Behälter als auch jegliche Behälterform mit senkrechten oder abgeböschten Wänden.

Bei z.B. rechteckigen oder polygonalen Formen des Gasspeichers ist es vorteilhaft, die vorgenannte Dimensionierungsvorschrift auch entlang weiterer Querschnitte anzuwenden. Bei einem rechteckigen Grundriss kann z.B. die Bogenlänge anhand eines Querschnitts in Richtung der Länge und der Breite bestimmt werden, um die Membran entsprechend anzupassen.

Bei einem Gasspeicher mit zwei oder mehreren Membranen sind alle Membranen so zu konfektionieren, dass sie im entleerten Zustand in erfindungsgemäßer Weise aufliegen können.

Nachfolgend wird die Erfindung anhand des in den beigeschlossenen Zeichnungen dargestellten Ausführungsbeispiels eingehend erläutert. Es zeigt dabei
Fig.1 eine Draufsicht auf ein Ausführungsbeispiel eines nicht erfindungsgemäßen Gasspeichers mit einer Membran.
Fig.2 einen Querschnitt entlang der Linie AA durch den Gasspeicher gemäß Fig.1;

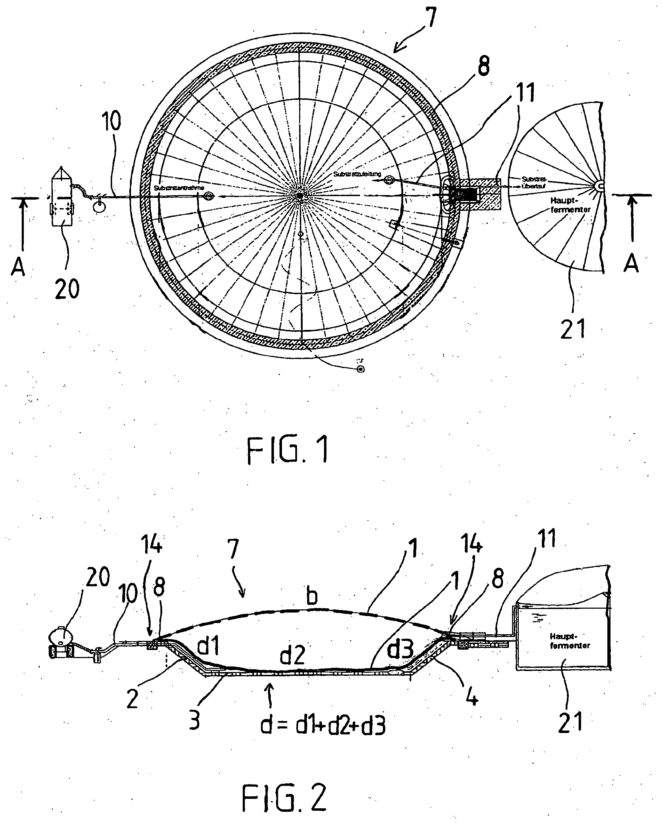

Fig.1 und 2 zeigen einen im Grundriß kreisrunden Gasspeicher 7 mit einem Speicherboden 3 und einer durch Wandschrägen 2, 4 gebildeten Begrenzungswand, wobei sowohl der Speicherboden 3 als auch die Wandschrägen 2, 4 mit einer nicht dargestellten Dichtungsschicht abgedeckt sind, um ein Durchsickern von Substrat zu verhindern.

An einer Beckenkrone 8 ist eine gasdichte Abdeckung in Form einer gasdichten Membran 1 durch Klemmung gasdicht festgelegt. Die gasdichte Membran 1 ist in Fig.2 sowohl in ihrer vollständig aufgespannten Stellung (strichlierte Linie) als auch in ihrer vollkommen abgesenkten Stellung (ausgezogene Linie) dargestellt.

Im Betrieb wird Substrat aus einem Hauptfermenter 21 über eine Substratleitung 11 in den Gasspeicher 7 geleitet und in diesem durch Fermentation Biogas erzeugt, wobei das sich bildende Biogas durch die gasdichte Membran 1 gegen ein Entweichen in die umgebende Atmosphäre gesichert ist. Bei ausreichender Produktion hebt sich die gasdichte Membran 1 in die strichliert eingezeichnete aufgespannte Stellung.

Noch während des Fermentationsvorgangs oder nach dessen Ende wird Gas über eine nicht dargestellte Leitung entnommen. Das verbrauchte Substrat kann über eine Ablassleitung 10 z.B. in ein Tankfahrzeug 20 umgepumpt werden. Bei der vollständigen Entleerung des Gasspeichers 7 sowohl hinsichtlich des Biogases als auch des Substrates kommt es zum Absinken der gasdichten Membran 1.

Das sonst übliche Gurtsystem zum Auflegen bzw. Abstützen der niedergesunkenen Membran 1 ist im gezeigten Ausführungsbeispiel nicht ausgebildet. Damit das Eigengewicht der gasdichten Membran 1 und gegebenenfalls auf dieser liegende Schneelasten keine Beschädigung der gasdichten Membran 1 verursachen, ist erfindungsgemäß vorgesehen, dass die gasdichte Membran 1 so konfektioniert ist, dass sie im entleerten Zustand des Gasspeichers 7 zumindest teilweise auf dem Speicherboden 3 und der Begrenzungswand 2, 4 flächig aufliegt (ausgezogene Linie). Die Membran ist gegen Beschädigungen geschützt, indem beim Aufliegen keine Spannungen in der Membran vorhanden sind, die zum Einreißen oder sonstiger Zerstörung führen könnten. Auch scharfkantige Gegenstände, wie Rührwerkschaufeln können somit keine Beschädigungen erzeugen und werden gegebenenfalls vor Absinken der Membran abgedeckt oder in eine entsprechende abgedeckte Position eingefahren.

Um ein spannungsfreies Aufliegen der gasdichten Membran nach dem Absinken zu ermöglichen, ist entlang dem in Fig.2 gezeigten Querschnitt durch den Gasspeicher 7 die Bogenlänge b der Membran 1 im aufgespannten Zustand größer oder gleich der zwischen gegenüberliegenden Einspannpunkten 14 der gasdichten Membran 1 gemessenen Länge der Durchlauflinie d entlang dem Speicherboden 3 (entsprechend dem Teilstück d2) und entlang der Schrägen der Begrenzungswand 2, 4 (entsprechend den Teilstücken d1 und d3).

Die Erfindung ist nicht auf eine kreisförmige Gestalt des Gasspeichers beschränkt, sondern es können auch andere, z.B. rechteckige, quadratische oder polygonale Grundrisse von Gasspeichern mit entsprechend konfektionierten gasdichten Membranen versehen sein, die auf dem Speicherboden und gegebenenfalls der Begrenzungswand abgelegt werden können.

Die Erfindung umfasst Erfindung umfasst den Gasspeicher-Typ mit zwei oder mehreren Membranen, z.B einer Innen- und einer Außenmembran. Im völlig entleerten Zustand liegen dann z.B. die Außenmembran und die Innenmembran auf dem Speicherboden und gegebenenfalls der Begrenzungswand auf. Die Art oder der Aufbau des Speicherbodens oder der Begrenzungswand unterliegen im Rahmen der Erfindung keiner Einschränkung. Es können somit auch Gasspeicher mit einer senkrecht vom Speicherboden wegstehenden Begrenzungswand mit der erfindungsgemäß dimensionierten Membran ausgestattet sein.

## Patentansprüche

1. Gasspeicher mit einem Speicherboden (3) und einer Begrenzungswand (2, 4) zur Aufnahme von Substrat, aus dem durch Fermentation Biogas erzeugbar ist, sowie einer gasdichten Abdeckung mit zumindest zwei, an ihrem Rand fixierten, gasdichten Membranen (1), die eine Innen- und eine Außenmembran umfassen, wobei die zumindest zwei gasdichten Membranen (1) so konfektioniert sind, dass sie im entleerten Zustand des Gasspeichers (7) zumindest teilweise auf dem Speicherboden (3) und der Begrenzungswand (2, 4) flächig aufliegen, **dadurch gekennzeichnet, dass** der Gasspeicher eine Substratleitung (11) umfasst, über die im Betrieb Substrat in den Gasspeicher geleitet werden kann.

2. Gasspeicher nach Anspruch 1, **dadurch gekennzeichnet, dass** zumindest entlang einem Querschnitt durch den Gasspeicher (7) die Bogenlänge b der zumindest einen Membran (1) im aufgespannten Zustand größer oder gleich der zwischen gegenüberliegenden Einspannpunkten (14) der zumindest zwei gasdichten Membranen (1) gemessenen Länge der Durchlauflinie d entlang dem Speicherboden (3) und gegebenenfalls der Begrenzungswand (2, 4) ist.

## Claims

1. A gas reservoir having a reservoir bottom (3) and a boundary wall (2, 4) for receiving substrate from which biogas is producible by fermentation, as well as a gastight cover having at least two gastight membranes (1) that are fastened at their edges and comprise one inner membrane and one outer membrane, said at least two gastight membranes (1) being manufactured in a way so that they at least partially rest two-dimensionally on the reservoir bottom (3) and the boundary wall (2, 4) when the gas reservoir (7) is empty, **characterized in that** said gas reservoir comprises a substrate conduit (11) via which substrate can be supplied into the gas reservoir when it is in use.

2. The gas reservoir according to claim 1, **characterized in that** at least along a cross-section through the gas reservoir (7), the arc length b of said at least one membrane (1) in a stretched state is greater than or equal to the length of the continuous line d measured between opposing clamping points (14) of said at least two gastight membranes (1) along the reservoir bottom (3) and the boundary wall (2, 4), if applicable.

## Revendications

1. Réservoir de gaz avec un fond (3) de réservoir et une paroi limite (2, 4) pour recevoir du substrat à partir duquel du biogaz peut être produit par fermentation, aussi bien qu'une couverture étanche au gaz avec au moins deux membranes (1) étanches au gaz fixées à leurs bords, comprenant une membrane intérieure et une membrane extérieure, lesdites au moins deux membranes (1) étanches au gaz étant confectionnées de sorte qu'elles reposent, au moins partiellement, en deux dimensions sur le fond (3) du réservoir et la paroi limite (2, 4) dans l'état vide du réservoir (7) de gaz, **caractérisé en ce que** le réservoir de gaz comprend une conduite de substrat (11), à travers laquelle du substrat peut être conduit dans le réservoir à gaz lors de son fonctionnement.

2. Réservoir de gaz selon la revendication 1, **caractérisé en ce qu'**au moins le long d'une coupe transversale du réservoir (7) de gaz, la longueur d'arc b de ladite au moins une membrane (1) dans un état étendu est supérieure ou égale à la longueur de la ligne continue d, mesurée entre des points de serrage (14) opposés desdites au moins deux membranes (1) étanches au gaz le long du fond (3) du réservoir et éventuellement de la paroi limite (2, 4).
